# EUROPEAN PATENT APPLICATION

(11) **EP 4 831 497 A2**
(43) Date of publication of application: **28.10.2026**
(21) Application number: 26190245.6
(22) Date of filing: 17.04.2019
(51) Int. Cl.: F16B 12/24

(54) **SYMMETRIC TONGUE & T-CROSS**

(30) Priority: 18.04.2018 SE 1850441
(62) Document number(s) of the earlier application(s) in accordance with Art. 76 EPC: 19789380.3 / 3 781 821
(71) Applicant: Välinge Innovation AB, 263 64 Viken (SE)
(72) Inventor: DERELÖV, Peter, SE-254 82 HELSINGBORG (SE)
(74) Representative: Välinge Innovation AB

(57) **Abstract**

A set comprising a first panel (1), a second panel (2) and a mechanical locking device for locking the first panel (1) to the second panel (2), wherein the first panel (1) comprises a first edge surface (11) and a first panel surface (12) and the second panel (2) comprises a second edge surface (21) and a second panel surface (22), characterized in that the mechanical locking device comprises an insertion groove (3) at the second edge surface (21), a flexible tongue (4) positioned in the insertion groove (3) and an edge tongue comprising a tongue groove (9), that the flexible tongue (4) comprises a first locking surface (101) and a second locking surface (102), that the first locking surface (101) is configured to cooperate with the tongue groove (9) for locking of the first panel (1) to the second panel (2) in a first direction when the flexible tongue (4) is positioned in the insertion groove (3) in a first orientation, and that the second locking surface (102) of the flexible tongue (4) is configured to cooperate with the tongue groove (9) for a locking of the first panel (1) to the second panel (2) in a first direction when the flexible tongue (4) is positioned in the insertion groove (3) in a second orientation.

## Description

### Technical Field

Embodiments of the present invention relate to panels configured to be locked together with a mechanical locking device. The panels may be floorboards to be locked together to obtain a floor product, or panels that may be assembled and locked together to obtain a furniture product, such as a bookshelf, a cupboard, a wardrobe, a box, a drawer or a furniture component and may thereafter be dismantled. The mechanical locking device may comprise a flexible tongue.

### Technical Background

A furniture product provided with a mechanical locking device is known in the art, as evidenced by WO2015/038059. The furniture product comprises a first panel connected perpendicular to a second panel by a mechanical locking device comprising a flexible tongue in an insertion groove.

The above description of various known aspects is the applicant's characterization of such, and is not an admission that any of the above description is considered as prior art.

Embodiments of the present invention address a need to provide panels that can be assembled and dismantled.

### Summary of the Invention

It is an object of certain aspects of the present invention to provide an improvement over the above described techniques and known art; particularly, to achieve a set that could be dismantled/disassembled after assembly without damaging the mechanical locking device, such that the set yet again could be assembled.

It is an object of at least certain embodiments and aspects of the present invention to provide an improvement over the above described techniques and known art.

A further object of at least certain aspects of the present invention is to facilitate assembling of panels configured to be assembled without the need of using any tools.

A further object of at least certain aspects of the present invention is to facilitate disassembling of panels configured to be assembled.

A further object of at least certain aspects of the present invention is to facilitate assembling and disassembling of panels configured to be assembled with a locking device that is easy to manufacture and to use.

A further object of at least certain aspects of the present invention is to facilitate a method of disassembling assembled panels.

A further object of at least certain aspects of the present invention is to facilitate assembling of panels configured to be assembled with a locking device that is easy to use and install and which reduces the risk of incorrect installation thereof.

A further object of at least certain aspects of the present invention is to facilitate assembling of panels configured to be assembled in a more stable and aesthetic way.

At least some of these and other objects and advantages that will be apparent from the description have been achieved by a set comprising a first panel, a second panel and a mechanical locking device for locking the first panel to the second panel, wherein the first panel comprises a first edge surface and a first panel surface and the second panel comprises a second edge surface and a second panel surface, characterized in that the mechanical locking device comprises an insertion groove at the second edge surface, a flexible tongue positioned in the insertion groove and an edge tongue comprising a tongue groove, that the flexible tongue comprises a first locking surface and a second locking surface, that the first locking surface is configured to cooperate with the tongue groove for locking of the first panel to the second panel in a first direction when the flexible tongue is positioned in the insertion groove in a first orientation, and that the second locking surface of the flexible tongue is configured to cooperate with the tongue groove for a locking of the first panel to the second panel in a first direction when the flexible tongue is positioned in the insertion groove in a second orientation.

The first locking surface and the second locking surface may be essentially symmetrically positioned on the flexible tongue.

An angle between the first and second locking surface may be within the range of about 90° to about 180°, preferably within the range of about 150° to about 175°, or preferably about 158°.

The flexible tongue may comprise a guiding surface configured to cooperate with the edge tongue during a displacement of the first panel relative to the second panel.

The guiding surface may in a first end be connected to the first locking surface and in a second end be connected to the second locking surface.

The guiding surface may have a curved shape.

The guiding surface may have the shape of a circular segment.

The curved shape of the guiding surface may have a radius between the first and second locking surfaces within the range of about 0,5 mm to about 3 mm, preferably within the range of 1 mm to 2 mm, or preferably about 1,5 mm.

The flexible tongue may have a thickness T and the curved shape of the guiding surface may have a radius between the first and second locking surfaces within the range of about 0,2xT to 1,2xT or about 0,4xT to about 0,8xT, or 0,6xT.

The guiding surface may be configured to cooperate with a curved shape surface of a disassembling tool, wherein the disassembling tool preferably has a circular cross section.

The flexible tongue may have a support surface positioned opposite to the guiding surface, wherein the support surface may have a curved shape with a radius within the range of about 25 mm to about 50 mm, preferably within the range of 30 mm to 45 mm, or preferably about 37 mm.

The curved shape of the support surface may have a radius that is smaller than a radius of a bottom surface of the insertion groove.

The first or second direction for locking of the first panel to the second panel may be parallel and/or perpendicular to the second panel surface.

The edge tongue may be a rod-shaped element at the first edge surface and at a third edge surface of the first panel and the mechanical locking device may comprise a first element groove at the second panel surface and a second element groove at a third panel surface of the second panel, wherein the rod-shaped element may comprise the tongue groove and may be configured to be inserted into the first or second element groove, wherein the first element groove may extend from the second panel surface to the insertion groove and the second element groove may extend from the third panel surface to the insertion groove, the first locking surface of the flexible tongue may be configured to cooperate with the tongue groove for a locking of the first panel to the second panel in a first direction which is perpendicular to the first panel surface when it is inserted into the first element groove and the first edge surface is arranged against the first panel surface; and the second locking surface of the flexible tongue may be configured to cooperate with the tongue groove for locking of the first panel to the second panel in a first direction which is perpendicular to the first panel surface when it is inserted into the second element groove and the third edge surface is arranged against the third panel surface.

A first crosscut of the rod-shaped element, in a plane parallel to the first or second panel surface, may have a circular shape or a rectangular shape.

The mechanical locking device may be configured to automatically lock the first panel to the second panel when the rod-shaped element is inserted into the first or second element groove and the first edge surface is arranged against the first panel surface and/or the third edge surface is arranged against the second panel surface.

The rod-shaped element may have a longitudinal shape with a length direction which may be parallel to the first panel surface of the first panel.

In one aspect the locking surface and the adjacent sides of the flexible tongue may preferably have edges that are rounded, i.e. have a radius, which results in that the tongue is easier to insert and move in the insertion groove, also reducing the risk for fibers falling off from the insertion groove when the flexible tongue is inserted and moved in the insertion groove.

According to one aspect there is provided a flexible tongue comprising a first locking surface and a second locking surface. The first locking surface is configured to cooperate with a tongue groove for locking of a first panel to a second panel in a first direction when the flexible tongue is positioned in an insertion groove in a first orientation. The second locking surface of the flexible tongue is configured to cooperate with the tongue groove for a locking of the first panel to the second panel in a first direction when the flexible tongue is positioned in the insertion groove in a second orientation.

The first locking surface and the second locking surface may be essentially symmetrically positioned on the flexible tongue.

An angle between the first and second locking surface may be within the range of about 90° to about 180°, preferably within the range of about 150° to about 175°, or preferably about 158°.

The flexible tongue may comprise a guiding surface.

The guiding surface may in a first end be connected to the first locking surface and in a second end be connected to the second locking surface.

The guiding surface may have a curved shape.

The guiding surface may have the shape of a circular segment.

The curved shape of the guiding surface may have a radius between the first and second locking surfaces within the range of about 0,5 mm to about 3 mm, preferably within the range of 1 mm to 2 mm, or preferably about 1,5 mm.

The flexible tongue may have a thickness T and the curved shape of the guiding surface may have a radius between the first and second locking surfaces within the range of about 0,2xT to 1,2xT or about 0,4xT to about 0,8xT or 0,6xT.

According to an aspect the core of the first panel and/or of the second panel may be a wood-based core, preferably made of MDF, HDF, OSB, WPC, plywood or particleboard. The core may also be a plastic core comprising thermosetting plastic or thermoplastic e.g. vinyl, PVC, PU or PET. The plastic core may comprise fillers.

The first panel and/or the second panel may also be of solid wood.

The second panel may also be of metal, such as sheet metal.

The first panel and/or the second panel may be provided with a decorative layer, such as a foil or a veneer, on one or more surfaces.

### Brief description of the drawings

These and other aspects, features and advantages of which embodiments of the invention are capable of, will be apparent and elucidated from the following description of embodiments and aspects of the present invention, reference being made to the accompanying drawings, in which
FIG. 1A shows a 3D-view of an embodiment of the invention during assembling.
FIG. 1B shows an embodiment of the flexible tongue according to an embodiment of the invention.
FIGS. 2A-2B show side views of an embodiment of the first panel according to an embodiment of the invention.
FIG. 2C shows an enlargement of part of the embodiment shown in FIG. 2B.
FIG. 3 shows an embodiment of the first panel and the second panel in a locked position according to an embodiment of the invention.
FIG. 4A shows a 3D-view of an embodiment of the invention during assembling.
FIG. 4B shows a cross-section of an embodiment of the second panel.
FIG. 5A shows a cross section of an embodiment of the first panel and the second panel in a locked position according to an embodiment of the invention.
FIG. 5B shows an enlargement of a part of the embodiment shown in FIG. 5A.
FIGS. 6A-6B show side views of an embodiment of the first panel and the second panel in a locked position according to an embodiment of the invention.
FIG. 7A shows a cross section of an embodiment of the first panel and the second panel in a locked position according to an embodiment of the invention.
FIG. 7B shows an enlargement of a part of the embodiment shown in FIG. 7A.
FIG. 8A shows a cross section of an embodiment of the first panel and the second panel in a locked position according to an embodiment of the invention.
FIGS. 8B-8C show an embodiment of the second panel according to an embodiment of the invention.
FIG. 8D shows a cross section of an embodiment of the first panel and the second panel during assembling and in a locked position according to an embodiment of the invention.
FIGS. 9A-9B show cross section of an embodiment of the first and the second panel, where a disassembling tool is used for disassembling of the first and second panel.
FIGS. 9C-9D show cross section of the first and second panel in a locked position and during assembling.
FIG. 10 shows a 3D-view of the first and second panel during assembling.
FIGS. 11A-11E shows different views of a flexible tongue according to an embodiment of the invention.
FIGS. 12A-12E shows different views of a flexible tongue according to an embodiment of the invention.
FIGS. 13A-13B show enlargements of a cross section A-A or B-B of part of the embodiments shown in FIGS. 11A-11E and 12A-12E.

### Detailed description

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The terminology used herein is for the purpose of describing particular aspects of the disclosure only, and is not intended to limit the disclosure. As used herein, the singular forms "a", "an" and "the" are intended to include the plural forms as well, unless the context clearly indicates otherwise.

It should be noted that the word "comprising" does not necessarily exclude the presence of other elements or steps than those listed and the words "a" or "an" preceding an element do not exclude the presence of a plurality of such elements. It should further be noted that any reference signs do not limit the scope of the claims, that the example aspects may be implemented at least in part by means of both hardware and software, and that several "means", "units" or "devices" may be represented by the same item of hardware.

The different aspects, alternatives and embodiments of the invention disclosed herein can be combined with one or more of the other aspects, alternatives and embodiments described herein. Two or more aspects can be combined.

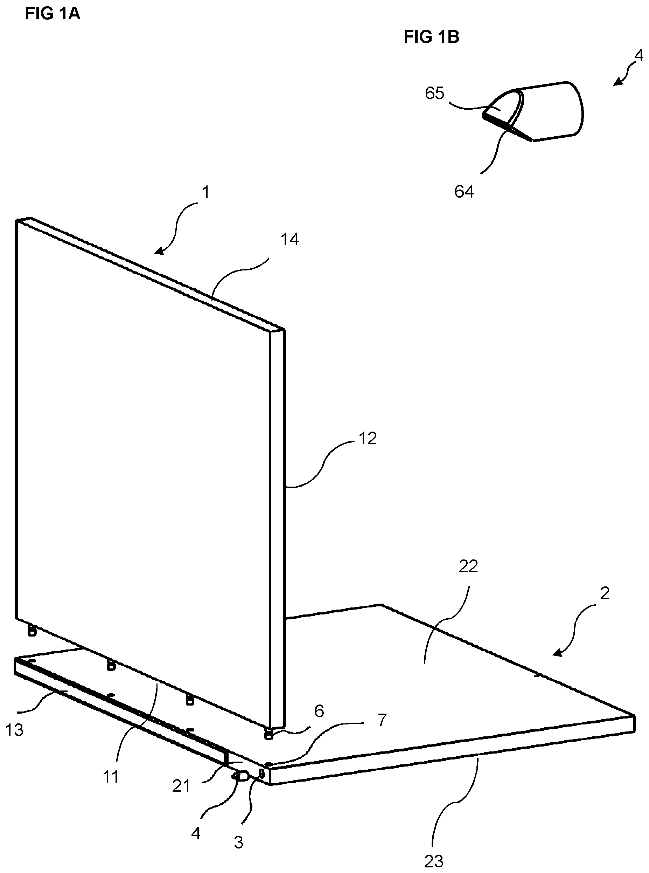

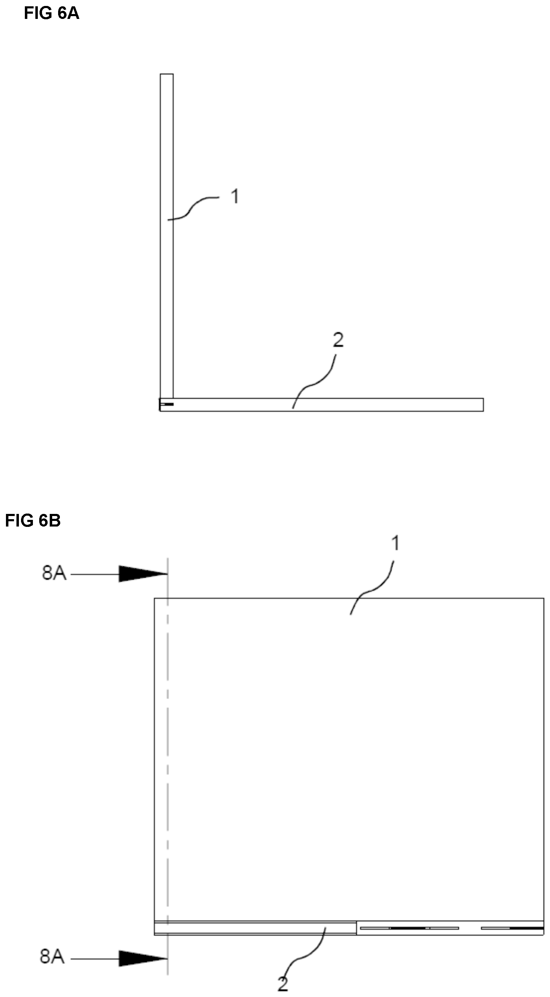

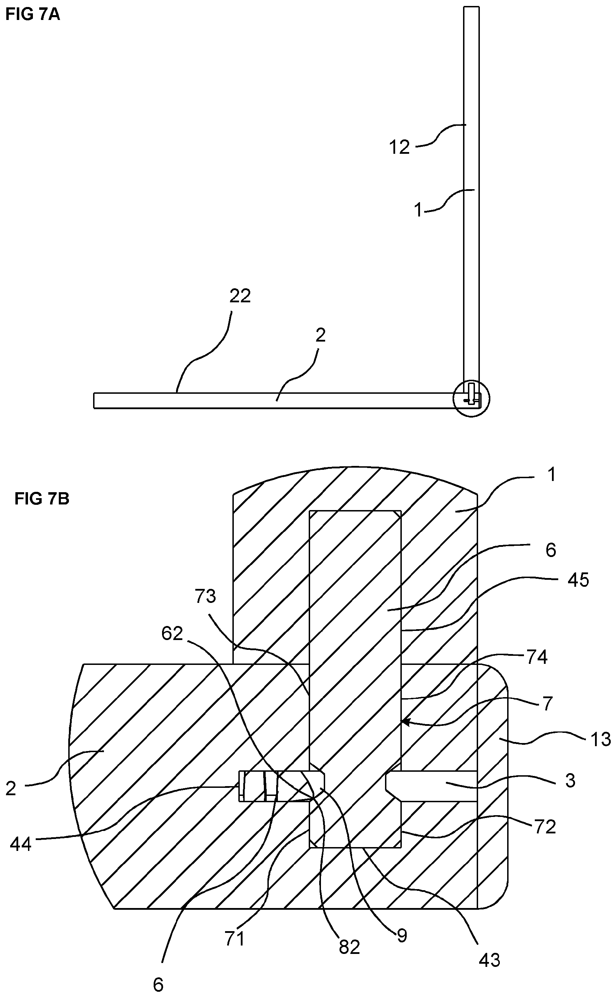

A first embodiment of the invention is shown, e.g., in FIGS 1-8A and 9A-13B including a set comprising a first panel 1, a second panel 2 and a mechanical locking device for locking the first panel 1 to the second panel 2. The first panel 1 comprises a first edge surface 11 and a first panel surface 12. The second panel 2 comprises a second edge surface 21 and a second panel surface 22. The mechanical locking device further comprises an insertion groove 3 at the second edge surface 21, a flexible tongue 4 positioned in the insertion groove 3 and an edge tongue comprising a tongue groove 9. The flexible tongue 4 comprises a first locking surface 101 and a second locking surface 102. The first locking surface 101 is configured to cooperate with the tongue groove 9 for locking of the first panel 1 to the second panel 2 in a first direction when the flexible tongue 4 is positioned in the insertion groove 3 in a first orientation. The second locking surface 102 of the flexible tongue 4 is configured to cooperate with the tongue groove 9 for a locking of the first panel 1 to the second panel 2 in a first direction when the flexible tongue 4 is positioned in the insertion groove 3 in a second orientation.

The first panel 1 and the second panel 2 are preferably panels for a furniture product and may be a part of a frame of a furniture product.

For a furniture product, the set is preferably configured for locking the first panel 1 to the second panel 2 with the first panel surface 12 perpendicular or essentially perpendicular to the second panel surface 22.

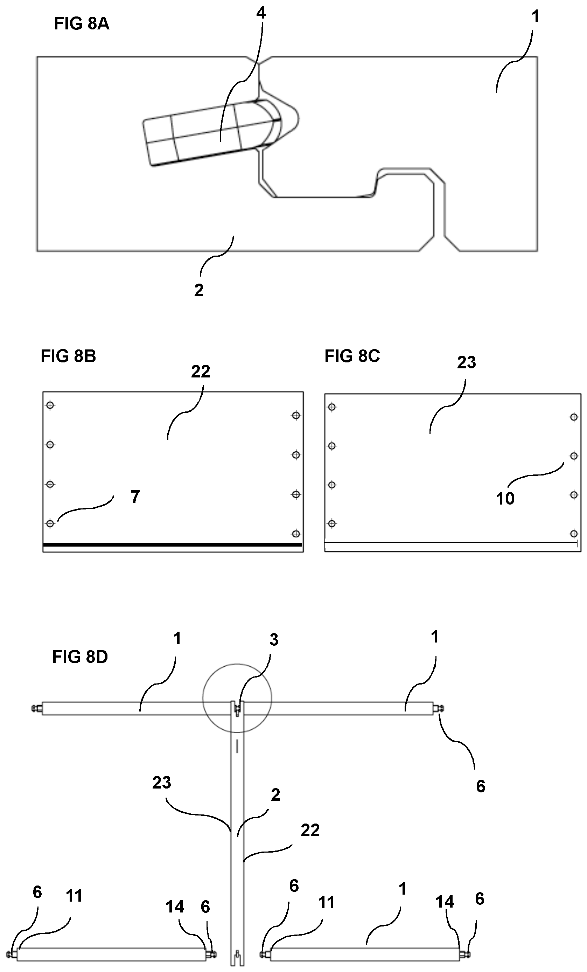

The first panel 1 and the second panel 2 may also be floorboards, as illustrated in FIG. 8A, to be locked together to obtain a floor product.

FIG 1 shows the first embodiment in a 3D view during assembling of a furniture product. The first panel may be assembled by displacing the first panel relative the second panel in a direction which is perpendicular to the second panel surface 22. The mechanical locking device may be configured to automatically lock the first panel 1 to the second panel 2 when the rod-shaped element 6 is inserted into the first element groove 7 and the first edge surface 11 is arranged against second panel surface 22. Embodiments are shown in WO 2018/080387 which are hereby expressly incorporated by reference.

An embodiment of the flexible tongue 4 is shown in FIG 1B in a 3D view which is configured for an insertion groove 3 which may comprise a circular cross section . The flexible tongue may have a curved surface which preferably matches the circular cross section of the edge groove 3. A first part of the flexible tongue is configured to cooperate with the insertion groove and a second part is configured to cooperate with the recess 9 of the rod-shaped element 6. An advantage with this embodiment of the flexible tongue is that the size of the edge groove may be small. A small edge groove may have the advantage that the effect of the strength of the second edge of the second panel is limited or non-existent.

The second part may comprise a first bevel 65 which configured to cooperate with the rod-shaped element 8 during assembling and a second bevel 64 which is configured to cooperate with the recess for the locking.

The flexible tongue may comprise a flexible material to enable compression and a displacement of the flexible tongue in the edge groove during assembling.

The flexible tongue may comprise an element which is flexible to enable compression and a displacement of the flexible tongue in the edge groove during assembling and another element which is less flexible in order to improve the locking strength.

A part of the curved surface of the flexible tongue 6 may be configured to be displaced against a surface, such as a cylindrical surface, of the edge groove 5.

The first edge surface 12 may comprise two or more of said rod shaped element and the second panel surface 3 may comprise two or more of said insertion groove 7, preferably arranged linearly, wherein each of the rod-shaped elements is configured to be inserted into one of the insertion grooves.

The first element groove 7 may be formed in the second panel surface 22 and in a core of the second panel 2.

The second panel surface 22 may comprise a decorative layer and the first element groove 7 may extend though the decorative layer.

The first element groove 7 may be formed by mechanical cutting, such as drilling.

An edge element 13, such as an edge band, is preferably attached to the second edge surface 21 for covering the insertion groove 3 and for reinforcing the second edge surface 21. The edge element 13 may be glued to the second edge surface 21 or attached by a mechanical locking device, which may comprise a part that protrudes from the edge element and is configured to be inserted into the insertion groove 3. The part may be attached to the insertion groove 3 by friction. The edge element 13 is in FIG 1A partly removed in order to visualize the flexible tongue 4 and the insertion groove 3. The flexible tongue 4 is shown before it is positioned in the insertion groove 3. The insertion groove 3 may comprise a circular cross section.

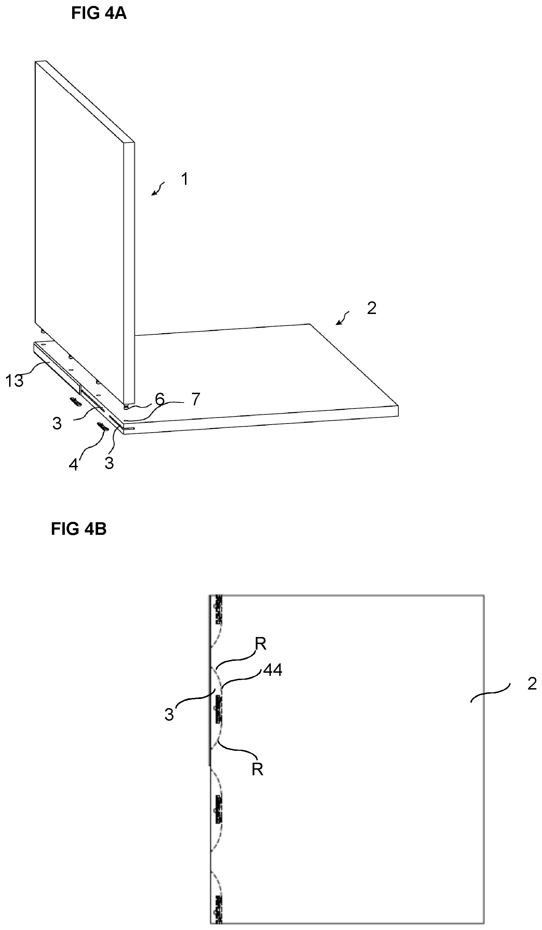

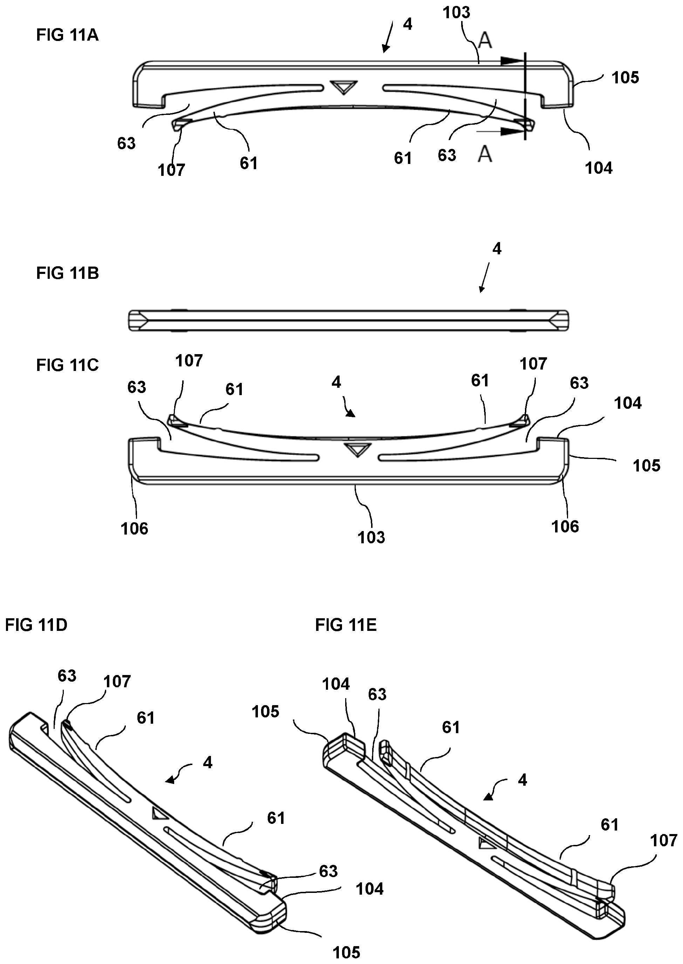

Embodiments are shown in FIGS. 4A-4B and 6A-10 which comprise an embodiment of the insertion groove 3 which is a longitudinal groove that extends in a longitudinal direction of the second edge surface 21. Embodiments of the flexible tongue 4 for a longitudinal groove are shown in FIGS. 11-13. The first locking surface 101 and the second locking surface 102 may be essentially symmetrically positioned on the flexible tongue 4. An angle 50 between the first 101 and second 102 locking surface is within the range of about 90° to about 180°, preferably within the range of about 150° to about 175°, or preferably about 158°.

The flexible tongue 4 may comprise a guiding surface 103 configured to cooperate with the edge tongue during a displacement of the first panel 1 relative to the second panel 2.

The guiding surface 103 in a first end may be connected to the first locking surface 101 and in a second end may be connected to the second locking surface 102.

The guiding surface 103 may have a curved shape.

The guiding surface 103 may have the shape of a circular segment.

The curved shape of the guiding surface 103 may have a radius between the first 101 and second 102 locking surfaces within the range of about 0,5 mm to about 3 mm, preferably within the range of 1 mm to 2 mm, or preferably about 1,5 mm.

The flexible tongue 4 may have a thickness T and the curved shape of the guiding surface 103 may have a radius between the first 101 and second 102 locking surfaces within the range of about 0,2xT to 1,2xT or about 0,4xT to about 0,8xT, or 0,6xT.

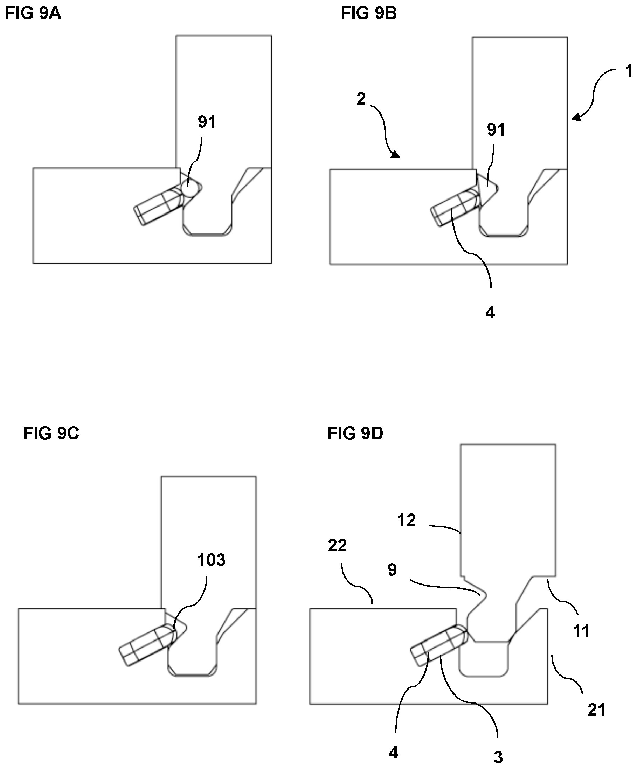

The guiding surface 103 may be configured to cooperate with a curved shape surface of a disassembling tool 91, wherein the disassembling tool may have a circular cross section, as shown in FIG 9A.

The guiding surface 103 may be configured to cooperate with a disassembling tool 91, wherein the disassembling tool may have a triangular cross section, as shown in FIG 9B.

The flexible tongue 4 may have a support surface 104 positioned opposite to the guiding surface 103, wherein the support surface 104 may have a curved shape with a radius within the range of about 25 mm to about 50 mm, preferably within the range of 30 mm to 45 mm, or preferably about 37 mm.

The curved shape of the support surface 104 may have a radius R which is smaller or the same as a radius of a bottom surface 44 of the insertion groove 3, as illustrated in Fig. 4B.

The flexible tongue 4 may comprise a bendable part 61, preferably a first bendable part and a second bendable part. The flexible tongue 4 preferably comprises a recess 63 at each of said bendable part. An advantage with this embodiment of the flexible tongue is that a stronger spring force may be obtained which may provide a stronger locking. A disadvantage for some embodiments of the second panel is that the size of the insertion groove 3 may have to be larger which may weaken the second edge surface 21 of the second panel 2.

The flexible tongue may comprise a side 106, with a beveled or rounded shape. The side 106 may be configured to guide the insertion tool 91 during insertion in to the tongue groove 9, see FIG 9B.

The flexible tongue may comprise a protruding friction element 107 on the first and/or second bendable part 61. The size of the friction element 107 does not have to be the same on the first and second bendable part, but the size of the friction element 107 may be bigger on one side, which facilitates the movement of the tongue 4 sideways in the insertion groove 3.

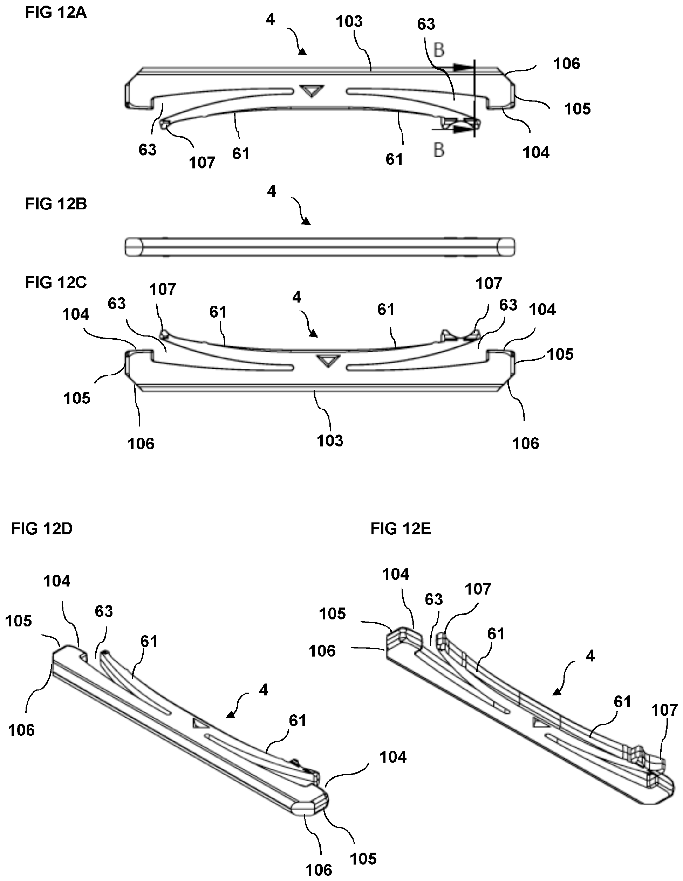

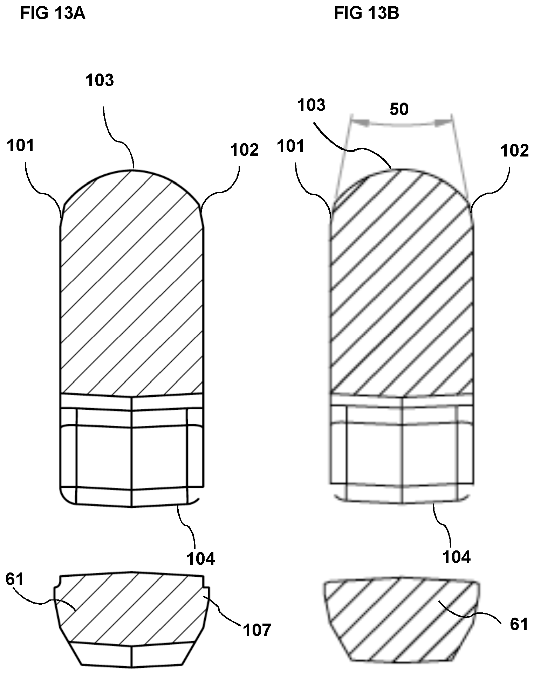

FIG 13A shows enlargements of a cross section A-A indicated FIG 11A. FIG 13B shows enlargements of a cross section B-B indicated FIG 12A. The cross section A-A extends through the protruding friction element 107. The cross section B-B is a side of the protruding friction element 107.

The first edge surface 11 may comprise two or more of said rod-shaped element 6 and the second panel surface 22 may comprise two or more of said first element groove 7, preferably arranged linearly, wherein each of the rod-shaped elements 6 is configured to be inserted into one of the first element grooves 7.

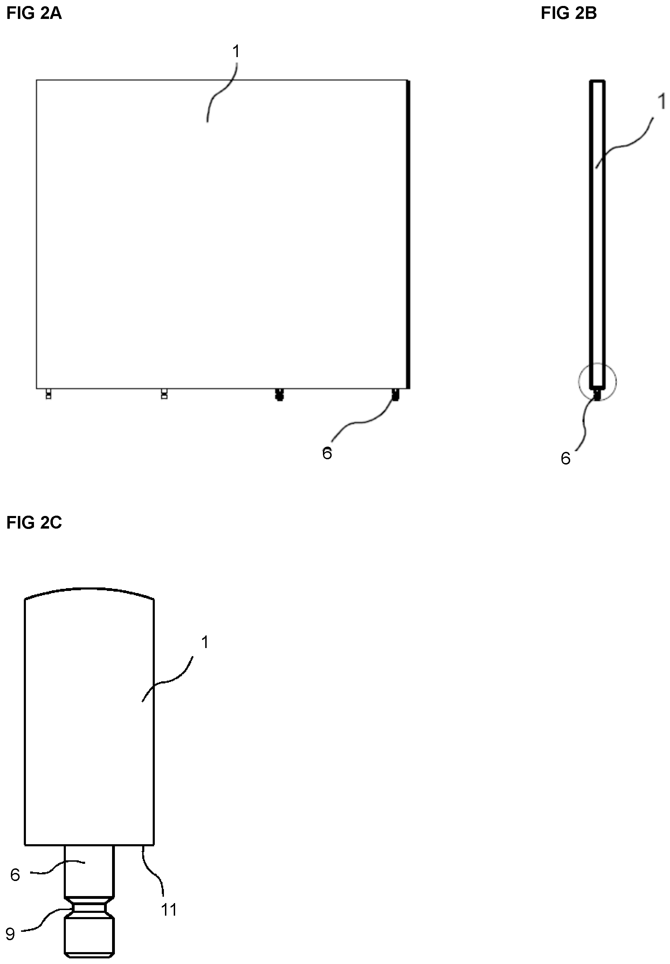

FIG 2A shows a first side view of the first panel 1 and FIG 2B shows a second side view of the first panel 1. FIG 2C shows an enlargement of the encircled area indicated in FIG 2B. An embodiment of the rod-shaped element 6 is shown which comprises an embodiment of the tongue groove 9. The rod-shaped element 6 has a longitudinal shape with a length direction which is parallel to the first panel surface. A first crosscut of the rod-shaped element 6, below and/or above the tongue groove 9, in a plane parallel to the second panel surface 22 may have a circular shape, a rectangular shape, a star shape, an oval shape or a hexagon shape.

A locking of the first panel 1 to the second panel 2 in a second direction which is perpendicular to the first panel surface 12 may be obtained by cooperating locking surfaces between the first element groove 7 and the rod-shaped element 6.

A locking of the first panel 1 to the second panel 2 in a third direction which is perpendicular to the first direction and the second direction surface may be obtained by cooperating locking surfaces between the first element groove 7 and the rod-shaped element 6.

A second cross cut of the first element groove 7, in a plane parallel to the second panel surface 22, preferably has a shape that matches a first cross cut of the rod-shaped element 6, in a plane parallel to the second panel surface 22. An advantage of this may be that an improved locking of the first panel 1 to the second panel 2 in the second direction is obtained and/or an improved locking of the first panel 1 to the second panel 2 in a third direction is obtained.

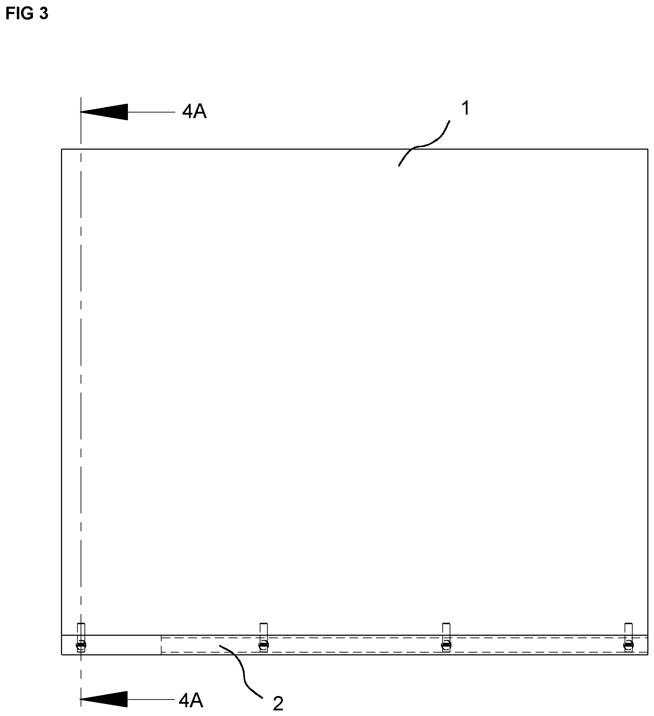

FIG 3 shows a side view of the first panel 1 and the second panel 2, for a furniture, in a locked position.

FIG 8A shows a cross cut of an embodiment of the first panel 1 and the second panel 2, for a floor product, in a locked position.

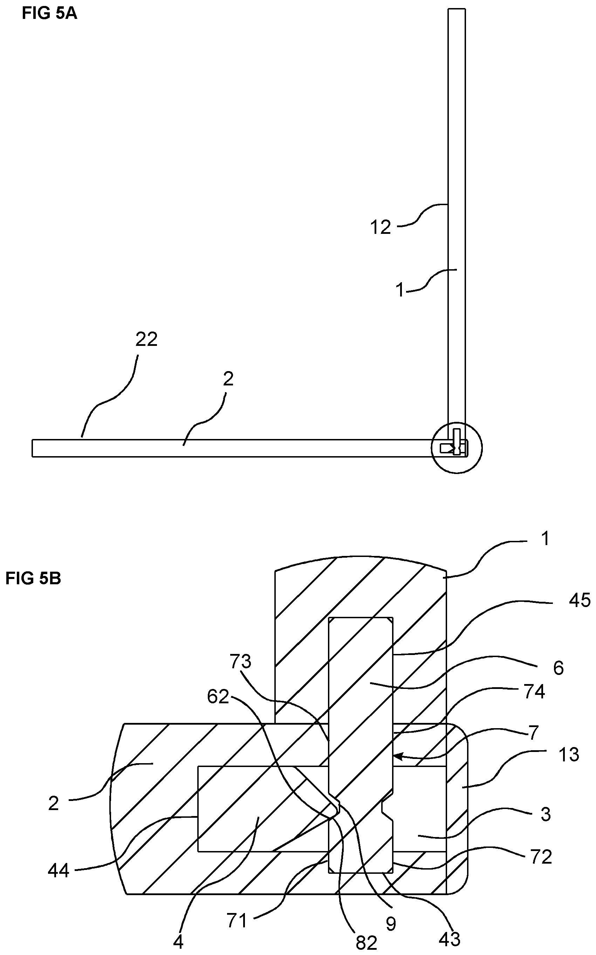

FIG 5A shows a cross cut, at the line indicated by 4A in FIG 3; of the first panel 1 and the second panel 2 in the locked position. FIG 5B shows an enlargement of the encircled area indicated in FIG 5A. The first element groove 7 may extend from the second panel surface 22 to the insertion groove 3. A locking surface 62 of the flexible tongue 4 may cooperate with a locking surface 82 of tongue groove 9 for the locking of the first panel to the second.

The first element groove 7 may comprise a bottom-ended groove, such as a bottom ended drill hole, comprising a bottom surface 43 which is positioned at a distance from the insertion groove 3.

The first element groove 7 may have a first part on a first side of the insertion groove 3 and a second part on a second side of the insertion groove 3, wherein the second part comprises a bottom surface 43 and side walls 71, 72, wherein, in a locked position, the rod-shaped element 6 passes through the first part of the first element groove 7, through the insertion groove 3 and into the second part of the first element groove 7.

The rod-shaped element 6 may be configured to cooperate, for the locking in the second direction, with the side walls 71, 72 of the second part of the first element groove 7.

The rod-shaped element 6 may be configured to cooperate, in a locked position, with the bottom surface 43, or may be configured not to cooperate, in a locked position, with the bottom surface, i.e. leaving a gap between the rod-shaped element 6 and the bottom surface 43.

The first part may comprise side walls 73, 74, wherein the rod-shaped element 6 may be configured to cooperate, for the locking in the second direction, with side walls 73, 74 of the first part.

The sidewalls may comprise material of the core of the second panel 2.

The insertion groove 3 may extend from the second edge surface 21 to the first element groove 7.

The insertion groove 3 may be a bottom-ended groove, comprising a bottom surface 44 which is positioned at a distance from the first element groove 7.

The flexible tongue 4 may be arranged at the bottom surface 44 of the insertion groove 3.

The flexible tongue 4 may be arranged between the tongue groove 9 and the bottom surface 44 of the insertion groove 3 in the locked position.

The rod-shaped element 6 may be configured to be attached in an attachment groove 45 in the first edge surface 11.

The rod-shaped element 6 may be configured to be glued in the attachment groove 45 in the first edge surface 11.

The rod-shaped element 6 may be configured to be locked in the attachment groove 45 by a friction connection or by a mechanically connection, such as threads or by a locking element, such as a barb.

The second edge surface 21 may be essentially perpendicular to the second panel surface 22.

An embodiment of the invention is shown in FIGS. 4A-B, 6A-7B and 9A-10, including a set comprising a first panel 1, a second panel 2 and a mechanical locking device for locking the first panel 1 to the second panel 2. The embodiment comprises an embodiment of the insertion groove 3 which is a longitudinal groove that extends in a longitudinal direction of the second edge surface 21. A locking surface 62 of the flexible tongue 4 may cooperate with a locking surface 82 of tongue groove 9 for the locking of the first panel to the second.

FIG. 9C shows an embodiment of the invention, where a first panel 1 is locked to a second panel 2. The flexible tongue 4 is inserted in the insertion groove 3 of the second panel 2, and the tongue groove 9 of the first panel cooperates with the first 101 or second 102 locking surface of the tongue 4 for locking of the first panel 1 to the second panel 2.

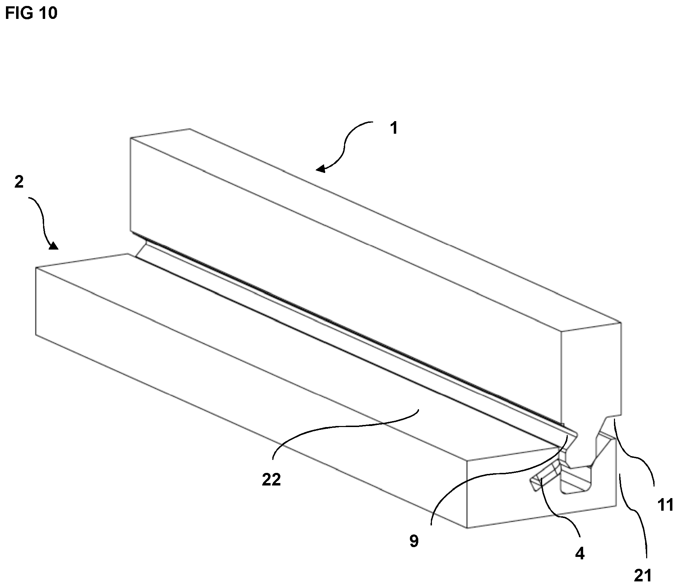

FIGS. 9D and 10 show an embodiment during assembling, where the embodiment as illustrated in 3D in FIG. 10 shows the locking device of first panel 1 made in one whole piece together with the panel 1, and that the tongue 4 is inserted in the insertion groove 3 of the second panel 2. This is an alternative to using the rod-shaped elements 6.

FIG 4A shows an embodiment in a 3D view during assembling. The first panel may be assembled by displacing the first panel relative the second panel 2 in a direction which is perpendicular to the second panel surface 22. The mechanical locking device may be configured to automatically lock the first panel 1 to the second panel 2 when the rod-shaped element 6 is inserted into the first element groove 7 and the first edge surface 11 is arranged against second panel surface 22.

The first element groove 7 may be formed in the second panel surface 22 and in a core of the second panel 2.

The second panel surface 22 may comprise a decorative layer and the first element groove 7 may extend though the decorative layer.

The first element groove 7 may be formed by mechanical cutting, such as milling or sawing.

An edge element 13, such as an edge band, is preferably attached to the second edge surface 21 for covering the insertion groove 3 and for enforcing the second edge surface 21. The edge element 13 may be glued to the second edge or attached by a mechanical locking device, which may comprise a part that protrudes from the edge element 13 and is configured to be inserted into the insertion groove 3. The part may be attached to the insertion groove 3 by friction. The edge element 13 is in FIG 4A partly removed in order to visualize the flexible tongue 4 and the insertion groove 3. The flexible tongue 4 is shown before it is positioned in the insertion groove 3.

In a second embodiment of the present invention, e.g., as shown in FIG. 8B-D, the edge tongue may be a rod-shaped element 6 at the first edge surface 11 and/or at a third edge surface 14, respectively, of the first panel 1 and the mechanical locking device may comprise a first element groove 7 at the second panel surface 22 and a second element groove 10 at a third panel surface 23 of the second panel 2. The rod-shaped element 6 may comprise the tongue groove 9 and may be configured to be inserted into the first 7 or second 10 element groove. The first element groove 7 may extend from the second panel surface 22 to the insertion groove 3 and the second element groove 10 may extend from the third panel surface 23 to the insertion groove 3. The first locking surface 101 of the flexible tongue 4 may be configured to cooperate with the tongue groove 9 for a locking of the first panel 1 to the second panel 2 in a first direction which is perpendicular to the first panel surface 22 when it is inserted into the first element groove 7 and the first edge surface 11 is arranged against the first panel surface 22. The second locking surface 102 of the flexible tongue 4 may be configured to cooperate with the tongue groove 9 for locking of the first panel 1 to the second panel 2 in a first direction which is perpendicular to the third panel surface 23 when it is inserted into the second element groove 10 and the third edge surface 14 is arranged against the third panel surface 23.

This allows for locking of several panels together, not just as one module, but several modules connected to each other, such as several cubes or rectangular.

As is illustrated in FIGS. 8B-8C, the position of the first 7 and second 10 element groove is displaced horizontally in relation to each other, and correspondingly the position of the rod-shaped element 6 at the first 11 and third 14 edge surface is displaced in relation to each other to fit in to the first 7 and second 10 element groove.

FIG 8D shows an embodiment which includes a second panel 2 comprising an embodiment of the insertion groove 3 (the encircled area in the drawing) with a flexible tongue 4. The flexible tongue 4 locks the first panel 1 to the second panel surface 22 of the second panel 2 and another of said first panel 1 to the third panel surface 23 of the second panel 2. The first locking surface 101 of the flexible tongue 4 cooperates with the tongue groove 9 of the rod-shaped element 6 at the first edge surface 11 of the first panel 1 and the second locking surface 102 of the flexible tongue 4 cooperates with the tongue groove 9 of the rod-shaped element 6 at the third edge surface 14 of said another first panel 1. Thus, one flexible tongue 4 may cooperate with two rod-shaped elements 6, one from each side of the tongue. In other embodiments, each tongue only cooperates with a single rod-shaped element 6.

The first panel 1 and the rod-shaped element 6 of the second embodiment may be identical to the first panel 1 and the rod-shaped element 6, respectively, of the first embodiment.

The features and characteristics as described above for the first element groove 7 also apply to the second element groove 10.

A first crosscut of the rod-shaped element 6, in a plane parallel to the first 22 or second 23 panel surface may have a circular shape or a rectangular shape.

The mechanical locking device may be configured to automatically lock the first panel 1 to the second panel 2 when the rod-shaped element 6 is inserted into the first 7 or second 10 element groove and the first edge surface 11 is arranged against the first panel surface 22 and/or the third edge surface 14 is arranged against the second 23 panel surface.

The rod-shaped element 6 may have a longitudinal shape with a length direction which is parallel to the first panel surface 12 of the first panel 1.

The insertion groove 3 may extend from the second edge surface 21 to the first element groove 7.

The insertion groove 3 may extend from the second edge surface to the second element groove 10.

The insertion groove 3 may comprise a first surface, an opposite second surface and a bottom surface 44 extending between the first surface and the opposite second surface.

The bottom surface 44 may be positioned at a distance from the first 7 and/or second 10 element groove.

The bendable part 61 of the flexible tongue 4 may be arranged at the bottom surface 44 of the insertion groove 3.

The flexible tongue 4 may be arranged at the bottom surface 44 of the insertion groove 3.

The flexible tongue 4 may be arranged between the tongue groove 9 and the bottom surface 44 of the insertion groove 3 in the locked position.

The support surface 104 and a side surface 105 of the flexible tongue 4 preferably have edges that are rounded to reduce the risk of fibers falling off from the insertion groove 3 when the flexible tongue 4 is inserted and moved in the insertion groove 3.

A part of the flexible tongue 4 may be configured to be displaced against a surface of the insertion groove 3, such as the first surface and/or the second surface.

The core of the first panel 1 and/or of the second panel 2 may be a wood-based core, preferably made of MDF, HDF, OSB, WPC, plywood or particleboard. The core may also be a plastic core comprising thermosetting plastic or thermoplastic e.g. vinyl, PVC, PU or PET. The plastic core may comprise fillers.

The first panel 1 and/or the second panel 2 may also be of solid wood.

The first panel 1 and/or the second panel 2 may be provided with a decorative layer, such as a foil or a veneer, on one or more surfaces.

The flexible tongue 4 according to the present invention enables a locking on both sides of the flexible tongue due to the first 101 and second 102 locking surface. Since the flexible tongue has locking surfaces on both sides, and thus may be symmetric, there risk of the tongue 4 being inserted in the insertion groove 3 in an incorrect way is strongly reduced.

Further, since the edges of the flexible tongue 4 are rounded, the risk of the flexible tongue 4 being stuck in the insertion groove 3 during the insertion of the flexible tongue 4 is reduced.

Further, the flexible tongue 4 according to an embodiment of the present invention enables the use of an essentially circular disassembling tool for disassembling of the panels. Such essentially circular disassembling tools are easier to manufacture with a high precision than rectangular disassembling tools. The circular disassembling tools further have a smaller cross-section than the rectangular ones, which has the advantage that a smaller opening in the panel for the disassembling tool is necessary, resulting in a more stable and aesthetic construction when the panels are mounted.

Further embodiments are disclosed below:
1. A set comprising a first panel 1, a second panel 2 and a mechanical locking device for locking the first panel 1 to the second panel 2, wherein the first panel 1 comprises a first edge surface 11 and a first panel surface 12 and the second panel 2 comprises a second edge surface 21 and a second panel surface 22, wherein the mechanical locking device comprises an insertion groove 3 at the second edge surface 21, a flexible tongue 4 positioned in the insertion groove 3 and an edge tongue comprising a tongue groove 9, wherein the flexible tongue 4 comprises a first locking surface 101 and a second locking surface 102, wherein the first locking surface 101 is configured to cooperate with the tongue groove 9 for locking of the first panel 1 to the second panel 2 in a first direction when the flexible tongue 4 is positioned in the insertion groove 3 in a first orientation, and wherein second locking surface 102 of the flexible tongue 4 is configured to cooperate with the tongue groove 9 for a locking of the first panel 1 to the second panel 2 in the first direction when the flexible tongue 4 is positioned in the insertion groove 3 in a second orientation.
2. The set as described in embodiment 1, wherein the first locking surface 101 and the second locking surface 102 is essentially symmetrically positioned on the flexible tongue 4.
3. The set as described in embodiment 1 or 2, wherein an angle 50 between the first 101 and second 102 locking surface is within the range of about 90° to about 180°, preferably within the range of about 150° to about 175°, or preferably about 158°.
4. The set as described in any one of the previous embodiments, wherein the flexible tongue 4 comprises a guiding surface 103 configured to cooperate with the edge tongue during a displacement of the first panel 1 relative to the second panel 2.
5. The set as described in embodiment 4, wherein the guiding surface 103 in a first end is connected to the first locking surface 101 and in a second end is connected to the second locking surface 102.
6. The set as described in embodiment 4 or 5, wherein the guiding surface 103 has a curved shape.
7. The set as described in embodiment 6, wherein the guiding surface 103 has the shape of a circular segment.
8. The set as described in embodiment 6 or 7, wherein the curved shape of the guiding surface 103 has a radius between the first 101 and second 102 locking surfaces within the range of about 0,5 mm to about 3 mm, preferably within the range of 1 mm to 2 mm, or preferably about 1,5 mm.
9. The set as described in anyone of the embodiments 6 to 8, wherein the flexible tongue has a thickness T and wherein the curved shape of the guiding surface 103 has a radius between the first 101 and second 102 locking surfaces within the range of about 0,2xT to 1,2xT or about 0,4xT to about 0,8xT, or 0,6xT.
10. The set as described in anyone of the embodiments 4 to 9, wherein the guiding surface 103 is configured to cooperate with a curved shape surface of a disassembling tool, wherein the disassembling tool preferably has a circular cross section.
11. The set as described in any one of the previous embodiments, wherein the flexible tongue 4 has a support surface 104 positioned opposite to the guiding surface 103, wherein the support surface 104 has a curved shape with a radius within the range of about 25 mm to about 50 mm, preferably within the range of 30 mm to 45 mm, or preferably about 37 mm.
12. The set as described in any one of embodiments 6 to 12, wherein the curved shape of the support surface 104 has a radius that is smaller than a radius of a bottom surface 44 of the insertion groove 3.
13. The set as described in any one of the previous embodiments, wherein the first or second direction for locking of the first panel 1 to the second panel 2 is parallel and/or perpendicular to the second panel surface 22.
14. The set as described in any one of the previous embodiments, wherein the edge tongue is a rod-shaped element 6 at the first edge surface 11 and at a third edge surface 14 of the first panel 1 and the mechanical locking device comprises a first element groove 7 at the second panel surface 22 and a second element groove 10 at a third panel surface 23 of the second panel 2, wherein the rod-shaped element 6 comprises the tongue groove 9 and is configured to be inserted into the first 7 or second 10 element groove, wherein
   the first element groove 7 extends from the second panel surface 22 to the insertion groove 3 and the second element groove 10 extends from the third panel surface 23 to the insertion groove 3,
   the first locking surface 101 of the flexible tongue 4 is configured to cooperate with the tongue groove 9 for a locking of the first panel 1 to the second panel 2 in a first direction which is perpendicular to the first panel surface 22 when it is inserted into the first element groove 7 and the first edge surface 11 is arranged against the first panel surface 22; and
   the second locking surface 102 of the flexible tongue 4 is configured to cooperate with the tongue groove 9 for locking of the first panel 1 to the second panel 2 in a first direction which is perpendicular to the first panel surface 22 when it is inserted into the second element groove 10 and the third edge surface 14 is arranged against the third panel surface 23.
15. The set as described in embodiment 14, wherein a first crosscut of the rod-shaped element 6, in a plane parallel to the first 22 or second 23 panel surface has a circular shape or a rectangular shape.
16. The set as described in any one of embodiments 14 to 15, wherein the mechanical locking device is configured to automatically lock the first panel 1 to the second panel 2 when the rod-shaped element 6 is inserted into the first 7 or second 10 element groove and the first edge surface 11 is arranged against the first panel surface 22 and/or the third edge surface 14 is arranged against the second 23 panel surface.
17. The set as described in any one of embodiments 14 to 16, wherein the rod-shaped element 6 has a longitudinal shape with a length direction which is parallel to the first panel surface 12 of the first panel 1.

## Claims

1. A set comprising a first panel (1), a second panel (2) and a mechanical locking device for locking the first panel (1) to the second panel (2), wherein the first panel (1) comprises a first edge surface (11) and a first panel surface (12) and the second panel (2) comprises a second edge surface (21 ) and a second panel surface (22), wherein the mechanical locking device comprises an insertion groove (3) at the second edge surface (21), a flexible tongue (4) positioned in the insertion groove (3) and an edge tongue comprising a tongue groove (9), wherein the flexible tongue (4) comprises a first locking surface (101) and a second locking surface (102), wherein the first locking surface (101) is configured to cooperate with the tongue groove (9) for locking of the first panel (1) to the second panel (2) in a first direction when the flexible tongue (4) is positioned in the insertion groove (3) in a first orientation of the flexible tongue (4), wherein the first direction is perpendicular to the second panel surface (22), wherein the second locking surface (102) of the flexible tongue (4) is configured to cooperate with the tongue groove (9) for a locking of the first panel (1) to the second panel (2) in the first direction when the flexible tongue (4) is positioned in the insertion groove (3) in a second orientation of the flexible tongue (4), wherein the first locking surface (101) and the second locking surface (102) is essentially symmetrically positioned on the flexible tongue (4) on both sides of the flexible tongue (4), thereby reducing the risk of the flexible tongue (4) being inserted in the insertion groove (3) in an incorrect way, wherein the flexible tongue (4) comprises a guiding surface (103) configured to cooperate with the edge tongue during a displacement of the first panel (1) relative to the second panel (2), and wherein the flexible tongue (4) is arranged at the bottom surface (44) of the insertion groove (3).

2. The set as claimed in claim 1, wherein the guiding surface (103) has a curved shape.

3. The set as claimed in claim 2, wherein the guiding surface (103) has the shape of a circular segment.

4. The set as claimed in claim 2 or 3, wherein the curved shape of the guiding surface (103) has a radius between the first (101) and second (102) locking surfaces within the range of about 0,5 mm to about 3 mm, preferably within the range of 1 mm to 2 mm, or preferably about 1 ,5 mm.

5. The set as claimed in anyone of the claims 1 to 4, wherein the flexible tongue has a thickness T and wherein the curved shape of the guiding surface (103) has a radius between the first (101) and second (102) locking surfaces within the range of about 0,2xT to 1,2xT or about 0,4xT to about 0,8xT, or 0,6xT.

6. The set as claimed in anyone of the previous claims, wherein the guiding surface (103) is configured to cooperate with a curved shape surface of a disassembling tool, wherein the disassembling tool preferably has a circular cross section.

7. The set as claimed in any one of the previous claims, wherein the flexible tongue (4) has a support surface (104) positioned opposite to the guiding surface (103), wherein the support surface (104) has a curved shape with a radius within the range of about 25 mm to about 50 mm, preferably within the range of 30 mm to 45 mm, or preferably about 37 mm.

8. The set as claimed in any one of the preceding claims, wherein a support surface (104), which is positioned opposite to the guiding surface, and a side surface (105) of the flexible tongue (4) have rounded edges.

9. The set as claimed in any one of the preceding claims, wherein the guiding surface (103) has a first end connected to the first locking surface (101) and a second end connected to the second locking surface (102).

10. The set as claimed in any one of the preceding claims, wherein the flexible tongue (4) comprises a first bendable part (61) and a second bendable part (61), and a recess (63) at each of the first and second bendable parts (61).

11. The set as claimed in claim 10, wherein the flexible tongue (4) comprises a protruding friction element (107) on at least one of the first and second bendable parts (61).

12. The set as claimed in any one of the preceding claims, wherein the first panel (1) and the second panel (2) are floorboards.

13. The set as claimed in any one of the claims 1-11, wherein the first panel (1) and the second panel (2) are panels for a furniture product

14. The set as claimed in claim 13, wherein the first panel (1) and the second panel (2) are of a frame of a furniture product.

15. The set as claimed in claims 13 or 14, wherein the mechanical locking device is configured for locking the first panel (1) to the second panel (2) with the first panel surface (12) perpendicular or essentially perpendicular to the second panel surface (22).
